# EUROPÄISCHE PATENTSCHRIFT

(11) **EP 4 106 841 B1**
(45) Veröffentlichungstag und Bekanntmachung des Hinweises auf die Patenterteilung: **13.08.2025**
(21) Anmeldenummer: 21702897.6
(22) Anmeldetag: 25.01.2021
(51) Int. Cl.: A61M 5/152

(54) **ELASTOMERE MEMBRAN FÜR EINE MEDIZINISCHE ELASTOMERPUMPE UND MEDIZINISCHE ELASTOMERPUMPE MIT EINER SOLCHEN ELASTOMEREN MEMBRAN**
ELASTOMERIC MEMBRANE FOR A MEDICAL ELASTOMERIC PUMP, AND MEDICAL ELASTOMERIC PUMP WITH SUCH AN ELASTOMERIC MEMBRANE
MEMBRANE ÉLASTOMÈRE POUR UNE POMPE ÉLASTOMÈRE MÉDICALE ET UNE POMPE ÉLASTOMÈRE MÉDICALE AVEC UNE TELLE MEMBRANE ÉLASTOMÈRE

(30) Priorität: 20.02.2020 DE 102020202186
(43) Veröffentlichungstag der Anmeldung: 28.12.2022
(73) Patentinhaber: B. Braun Melsungen AG, 34212 Melsungen (DE)
(72) Erfinder: WILDHAGEN, Jens, 30659 Hannover (DE); RIEMANN, Niklas, 36211 Alheim (DE)
(74) Vertreter: Patentanwälte Ruff, Wilhelm, Beier, Dauster & Partner mbB
(86) Internationale Anmeldenummer: PCT/EP2021/051572
(87) Internationale Veröffentlichungsnummer: WO 2021/164986

(56) Entgegenhaltungen:
- CN-U- 204 485 050
- DE-A1- 102018 211 140
- US-A- 5 298 025
- US-A- 5 368 570

## Beschreibung

Die Erfindung betrifft eine elastomere Membran für eine medizinische Elastomerpumpe zur Förderung einer medizinischen Flüssigkeit, wobei die elastomere Membran ein Pumpvolumen zur Aufnahme und Förderung der medizinischen Flüssigkeit ausbildet und in einem wenigstens teilweise mit der medizinischen Flüssigkeit befüllten Füllzustand des Pumpvolumens elastisch gedehnt ist, wodurch die elastisch gedehnte elastomere Membran einen Förderdruck auf das Pumpvolumen zur Förderung der medizinischen Flüssigkeit bewirkt. Die Erfindung betrifft zudem eine medizinische Elastomerpumpe mit einer solchen elastomeren Membran.

Eine derartige medizinische Elastomerpumpe ist aus der DE 10 2017 205 251 A1 (sowie US 5 368 570 A und US 5 298 025 A) bekannt und zur Verabreichung einer medizinischen Flüssigkeit an einen Patienten im Rahmen einer ambulanten oder stationären Infusionstherapie vorgesehen. Solche medizinischen Elastomerpumpen können auch als elastomerische Infusionspumpen bezeichnet werden. Die bekannte Elastomerpumpe weist eine elastomere Membran auf, die ein Pumpvolumen ausbildet, das zur Aufnahme und Förderung der zu verabreichenden medizinischen Flüssigkeit dient. In einem wenigstens teilweise mit der medizinischen Flüssigkeit befüllten Füllzustand des Pumpvolumens ist die elastomere Membran ballonartig elastisch gedehnt und nimmt eine in etwa kugelige oder eiförmige Gestalt ein. Die elastische Dehnung der elastomeren Membran bewirkt einen Förderdruck auf die in dem Pumpvolumen befindliche medizinische Flüssigkeit. Unter Einwirkung des Förderdrucks ist die medizinische Flüssigkeit ausgehend von dem Pumpvolumen in ein der elastomeren Membran flussabwärts nachgelagertes Fluidleitungssystem förderbar. Dabei ist eine sich hierbei einstellende Förderrate der medizinischen Flüssigkeit abhängig vom Förderdruck. Dieser ist abhängig von der elastischen Dehnung der elastomeren Membran, welche wiederum vom Füllzustand des Pumpvolumens und damit von der Füllmenge der in der elastomeren Membran befindlichen medizinischen Flüssigkeit abhängt. Infolge des füllzustandsabhängigen Förderdrucks stellt sich eine über dem Füllzustand und damit auch der Verabreichungsdauer veränderliche Förderrate der medizinischen Flüssigkeit ein. Aus medizinischer Sicht ist eine möglichst konstante Förderrate wünschenswert. Im Stand der Technik sind Drosselelemente in Form von Druckminderern oder Förderratenbegrenzern bekannt, die einer Verstetigung der Förderrate über dem Füllzustand dienen sollen und üblicherweise flussabwärts der elastomeren Membran angeordnet sind.

Aufgabe der Erfindung ist es, eine elastomere Membran und eine medizinische Elastomerpumpe der eingangs genannten Art bereitzustellen, die eine möglichst konstante Förderrate ermöglichen.

Diese Aufgabe wird für die elastomere Membran dadurch gelöst, dass die elastomere Membran mehrere Ausbuchtungsabschnitte aufweist, die wenigstens im Füllzustand unter Ausbildung jeweils einer Ausbuchtung ausgewölbt sind, wobei die Ausbuchtungen jeweils einen Pumpvolumenabschnitt des Pumpvolumens bilden. Es hat sich gezeigt, dass durch die erfindungsgemäße Lösung eine verringerte Abhängigkeit des Förderdrucks vom Füllzustand und damit auch von der Förderdauer erreicht werden kann. Hierdurch kann die medizinische Flüssigkeit mit einer möglichst konstanten Förderrate gefördert werden, was aus medizinischer Sicht wünschenswert und einer Patientensicherheit zuträglich ist. Durch die erfindungsgemäße Lösung ist das Pumpvolumen gleichsam in mehrere Pumpvolumenabschnitte untergliedert. Hierfür weist die elastomere Membran die mehreren Ausbuchtungsabschnitte auf. Die Ausbuchtungsabschnitte bilden in dem wenigstens teilweise mit der medizinischen Flüssigkeit befüllten Zustand der elastomeren Membran jeweils eine der Ausbuchtungen. Diese bilden wiederum jeweils einen der Pumpvolumenabschnitte des Pumpvolumens. Im Ergebnis wird durch die erfindungsgemäße Lösung - vereinfacht ausgedrückt - eine Art hydraulische Parallelschaltung mehrerer Pumpvolumina, nämlich der Pumpvolumenabschnitte, erreicht. Die Erfinder haben erkannt, dass durch diese Art hydraulische Parallelschaltung mit einfachen Mitteln eine Verstetigung des Förderdrucks und damit auch der Förderrate erreicht werden kann. Die elastomere Membran ist elastisch, vorzugsweise weich- und/oder gummielastisch, nach Art eines Ballons oder einer Blase dehnbar. Zu diesem Zweck ist die elastomere Membran aus wenigstens einem elastomeren Werkstoff gefertigt. Als elastomerer Werkstoff kommen insbesondere Silikon in Form von Silikonkautschuk oder Silikonelastomer, Gummi oder dergleichen infrage. In dem wenigstens teilweise mit der medizinischen Flüssigkeit befüllten Füllzustand ist die elastomere Membran ballon- oder blasenartig elastisch, vorzugsweise weich- und/oder gummielastisch, gedehnt und umgrenzt das Pumpvolumen und die darin aufgenommene medizinische Flüssigkeit. Infolge der erfindungsgemäß vorgesehenen Ausbuchtungsabschnitte nimmt die elastomere Membran - wenigstens im Füllzustand - nicht etwa eine kugel-, ei- oder ballförmige Gestalt ein. Vielmehr bilden die Ausbuchtungsabschnitte jeweils eine der Ausbuchtungen. Die Ausbuchtungen sind jeweils, vorzugsweise rundlich, ausgewölbt und können insbesondere auch als Rundung, Wölbung, Schwellung, Bauch oder Buckel bezeichnet werden. Die Ausbuchtungen können jeweils insbesondere kugelkalottenförmig ausgewölbt sein. Demgemäß weist die elastomere Membran - wenigstens im Füllzustand - eine an mehreren Stellen, nämlich den Ausbuchtungen, ausgerundete, ausgewölbte, geschwollene und/oder bauchige Gestalt auf. Entsprechendes kann für einen nicht mit der medizinischen Flüssigkeit befüllten Leerzustand der elastomeren Membran gelten. Alternativ sind die Ausbuchtungen im Leerzustand nicht und/oder in einer geringeren Ausprägung ausgebildet. Die Ausbuchtungsabschnitte und damit auch die Ausbuchtungen sind vorzugsweise gleichmäßig über die Oberfläche der elastomeren Membran verteilt angeordnet. Die Ausbuchtungsabschnitte können eine gleichförmige, bevorzugt eine identische, oder ungleichförmige Formgebung aufweisen.

In Ausgestaltung der Erfindung weist die elastomere Membran örtlich unterschiedliche elastische Dehnungseigenschaften auf, wobei die elastomere Membran im Bereich der Ausbuchtungsabschnitte vergleichsweise leichter elastisch dehnbar ist als abseits der Ausbuchtungsabschnitte. Durch die vergleichsweise leichtere elastische Dehnbarkeit im Bereich der Ausbuchtungsabschnitte ist gewährleistet, dass die elastomere Membran bei einem Befüllen mit der medizinischen Flüssigkeit und/oder im Füllzustand funktionsgerecht und zuverlässig im Bereich der Ausbuchtungsabschnitte rundlich auswölbt und somit die Ausbuchtungen ausbildet. Die örtlich unterschiedlichen elastischen Dehnungseigenschaften können werkstoffseitig und/oder durch eine entsprechende Dimensionierung erreicht sein. Beispielsweise kann die elastomere Membran im Bereich der Ausbuchtungsabschnitte aus einem ersten Werkstoff und abseits hiervon aus einem zweiten Werkstoff gefertigt sein, wobei der erste Werkstoff im Vergleich zu dem zweiten Werkstoff einen geringeren Elastizitätsmodul aufweist. Alternativ oder zusätzlich kann die elastomere Membran durchgängig aus ein- und demselben Werkstoff gefertigt sein, wobei dieser einen örtlich unterschiedlichen Vernetzungsgrad aufweisen kann. Weiter alternativ oder zusätzlich kann die elastomere Membran im Bereich der Ausbuchtungsabschnitte schwächer dimensioniert sein, so dass eine vergleichsweise leichtere elastische Dehnbarkeit erreicht wird.

In weiterer Ausgestaltung der Erfindung weisen die Ausbuchtungsabschnitte unterschiedliche elastische Dehnungseigenschaften auf, wodurch die Ausbuchtungen unter Einwirkung des Förderdrucks unterschiedlich ausgebildet sind. Infolge der unterschiedlichen elastischen Dehnungseigenschaften sind die Ausbuchtungsabschnitte im Füllzustand unterschiedlich rundlich ausgewölbt. Hierdurch bilden die entsprechenden Ausbuchtungen Pumpvolumenabschnitte mit unterschiedlichem Volumeninhalt. Die Erfinder haben erkannt, dass eine solche hinsichtlich der elastischen Dehnungseigenschaften unterschiedliche Gestaltung der Ausbuchtungsabschnitte weitere Vorteile im Hinblick auf die angestrebte möglichst konstante Förderrate der medizinischen Flüssigkeit bietet. Die unterschiedlichen elastischen Dehnungseigenschaften der Ausbuchtungsabschnitte können werkstoffseitig und/oder durch eine entsprechende Dimensionierung erreicht sein.

In weiterer Ausgestaltung der Erfindung weist die elastomere Membran örtlich unterschiedliche Membrandicken auf, wobei eine erste Membrandicke im Bereich der Ausbuchtungsabschnitte vergleichsweise geringer ist als eine zweite Membrandicke abseits der Ausbuchtungsabschnitte. Durch die im Bereich der Ausbuchtungsabschnitte vergleichsweise geringere erste Membrandicke ist gewährleistet, dass die elastomere Membran unter Einwirkung der medizinischen Flüssigkeit funktionsgerecht auswölbt und die Ausbuchtungen ausbildet. Denn infolge der vergleichsweise geringeren ersten Membrandicke ist die elastomere Membran im Bereich der Ausbuchtungsabschnitte leichter elastisch dehnbar als abseits der Ausbuchtungsabschnitte. Dort ist die vergleichsweise stärker bemessene zweite Membrandicke vorgesehen. Sofern die elastomere Membran mittels eines Spritzgießverfahrens hergestellt wird, können ein hierbei verwendeter Werkstoff zur Fertigung der elastomeren Membran in einer örtlich unterschiedlichen Menge eingespritzt und somit unterschiedliche Membrandicken ausgebildet werden. Alternativ oder zusätzlich kann die elastomere Membran zur Ausbildung der örtlich unterschiedlichen Membrandicken materialabtragend bearbeitet sein.

In weiterer Ausgestaltung der Erfindung weisen die Ausbuchtungsabschnitte unterschiedliche Membrandicken auf. Hierdurch können unterschiedliche elastische Dehnungseigenschaften der Ausbuchtungsabschnitte erreicht werden. Beispielsweise kann ein erster Ausbuchtungsabschnitt der Ausbuchtungsabschnitte die vorgenannte erste Membrandicke aufweisen. Ein zweiter Ausbuchtungsabschnitt der Ausbuchtungsabschnitte kann eine hierzu vergleichsweise geringere oder stärker bemessene weitere, insbesondere dritte, Membrandicke aufweisen. Hinsichtlich der Ausbildung der unterschiedlichen Memrandicken wird auf die Offenbarung zu der vorherigen Ausgestaltung der Erfindung verwiesen. Das dort diesbezüglich Gesagte gilt im Hinblick auf diese Ausgestaltung der Erfindung sinngemäß.

In weiterer Ausgestaltung der Erfindung ist die elastomere Membran aus wenigstens einem elastomeren Werkstoff gefertigt, der örtlich unterschiedlich stark vernetzt ist, wobei ein erster Vernetzungsgrad im Bereich der Ausbuchtungsabschnitte vergleichsweise schwächer ist als ein zweiter Vernetzungsgrad abseits der Ausbuchtungsabschnitte. Durch die örtlich unterschiedlich starke Vernetzung des wenigstens einen elastomeren Werkstoffs weist dieser örtlich unterschiedliche elastische Dehnungseigenschaften auf. Dabei geht eine vergleichsweise schwache Vernetzung bekanntermaßen mit einer vergleichsweise leichten elastischen Dehnbarkeit einher und umgekehrt. Die örtlich unterschiedlich starke Vernetzung kann fertigungsseitig beispielsweise mittels einer örtlich unterschiedlich ausgeprägten Vulkanisation erreicht werden. Die Vulkanisation kann mittels hierfür grundsätzlich bekannter Verfahren erreicht werden, insbesondere einer Schwefelvulkanisation, einer Vulkanisation mittels Peroxiden, Metalloxiden oder energiereicher Strahlung.

In weiterer Ausgestaltung der Erfindung sind im Bereich der Ausbuchtungsabschnitte unterschiedliche Vernetzungsgrade vorgesehen. Durch die unerschiedlichen Vernetzungsgrade können unterschiedliche elastische Dehnungseigenschaften der Ausbuchtungsabschnitte erreicht werden. Beispielsweise kann ein erster Ausbuchtungsabschnitt der Ausbuchtungsabschnitte den vorgenannten ersten Vernetzungsgrad aufweisen. Ein zweiter der Ausbuchtungsabschnitte kann einen vergleichsweise schwächeren oder stärkeren, weiteren, insbesondere dritten, Vernetzungsgrad aufweisen. Bezüglich der fertigungsseitigen Maßnahmen zur Ausbildung der unterschiedlichen Vernetzungsgrade wird auf die vorherige Ausgestaltung der Erfindung verwiesen. Das dort diesbezüglich Gesagte gilt vorliegend sinngemäß.

In weiterer Ausgestaltung der Erfindung weist die Membran zwischen 2 und 100, bevorzugt zwischen 7 und 40, besonders bevorzugt zwischen 15 und 25, Ausbuchtungsabschnitte auf. Eine Anzahl zwischen 2 und 100 Ausbuchtungsabschnitten ist praktisch für sämtliche infrage kommenden Anwendungsfälle und damit auch Größen des Pumpvolumens vorteilhaft. Eine Anzahl zwischen 7 und 40 Ausbuchtungsabschnitten ist vergleichsweise bevorzugt. Denn hierdurch ist die maximale Anzahl der Ausbuchtungsabschnitte vergleichsweise geringer, so dass eine vergleichsweise vereinfachte Fertigung unter gleichzeitiger Beibehaltung einer vorteilhaften Verstetigung der Förderrate erreicht werden kann. Eine Anzahl zwischen 15 und 25 Ausbuchtungsabschnitten ist vergleichsweise besonders bevorzugt. Denn insbesondere in Bezug auf die angestrebte Verstetigung der Förderrate einerseits und eine möglichst einfache Gestaltung der elastomeren Membran andererseits wird hierdurch ein Optimum erreicht.

In weiterer Ausgestaltung der Erfindung sind eine Membrandicke und/oder örtlich unterschiedliche Membrandicken zwischen 0,5 mm und 4 mm, bevorzugt zwischen 1,5 mm und 2,5 mm, besonders bevorzugt zwischen 1,7 mm und 1,9 mm, vorgesehen. Durch den Bereich zwischen 0,5 mm und 4 mm werden nahezu sämtliche praktischen Anwendungsfälle abgedeckt. Der Bereich zwischen 1,5 mm und 2,5 mm ist bevorzugt, da hierdurch einerseits durch die vergleichsweise Reduzierung der maximalen Membrandicke eine Materialeinsparung und andererseits gleichzeitig ein zuverlässiges Auswölben der Ausbuchtungsabschnitte erreicht werden kann. Als diesbezüglich besonders vorteilhaft hat sich der Bereich zwischen 1,7 mm und 1,9 mm erwiesen.

In weiterer Ausgestaltung der Erfindung weist die elastomere Membran einen wenigstens einschichtigen Membranaufbau auf, der wenigstens eine aus Silikon gefertigte erste Membranschicht aufweist. Das Silikon kann insbesondere ein Silikonkautschuk oder ein Silikonelastomer sein. Der einschichtige Membranaufbau ermöglicht eine besonders einfache und damit kostengünstige Fertigung der elastomeren Membran, wodurch Kosten eingespart werden können. Durch die Verwendung von Silikon als erste Membranschicht können insbesondere vorteilhafte chemische Eigenschaften der elastomeren Membran erreicht werden.

In weiterer Ausgestaltung der Erfindung weist die elastomere Membran einen mehrschichtigen Membranaufbau auf, der wenigstens eine aus Gummi gefertigte zweite Membranschicht aufweist. Der mehrschichtige Membranaufbau ermöglicht eine vorteilhafte Anpassbarkeit der Eigenschaften der elastomeren Membran insbesondere in chemischer und/oder mechanischer Hinsicht. Vorzugsweise dominiert die zweite Membranschicht die mechanischen Eigenschaften der elastomeren Membran. Die in Bezug auf eine Wechselwirkung mit der medizinischen Flüssigkeit maßgeblichen chemischen Eigenschaften der elastomeren Membran werden vorzugsweise von der ersten Membranschicht dominiert. Dementsprechend ist vorzugsweise die erste Membranschicht dem Pumpvolumen zugewandt und als radial innenliegende Schicht vorgesehen. Die zweite Membranschicht ist vorzugsweise in radialer Richtung außenliegend und weiter vorzugsweise unmittelbar auf der ersten Membranschicht angeordnet. Es versteht sich, dass weitere Membranschichten vorgesehen sein können.

Die Erfindung betrifft zudem eine Membrananordnung für eine medizinische Elastomerpumpe zur Förderung einer medizinischen Flüssigkeit, mit einer elastomeren Membran nach der vorstehenden Beschreibung oder der eingangs genannten Art und mit einer die elastomere Membran wenigstens abschnittsweise umhüllenden dehnfesten Gitterstruktur, an deren radial innenliegender Innenseite die elastomere Membran wenigstens im Füllzustand radial nach außen abgestützt ist, wobei die Gitterstruktur mehrere Gitteröffnungen aufweist, durch welche die Ausbuchtungsabschnitte wenigstens im Füllzustand unter Ausbildung jeweils einer der Ausbuchtungen radial ausgewölbt sind. Die Gitterstruktur bildet eine Art Umhüllung der elastomeren Membran, wobei die elastomere Membran - wenigstens in teilweise mit der medizinischen Flüssigkeit befülltem Füllzustand - durch die mehreren Gitteröffnungen der Gitterstruktur radial nach außen ausgewölbt ist. Im Unterschied zu der elastomeren Membran ist die Gitterstruktur dehnfest ausgebildet. Bei einem Befüllen der elastomeren Membran und damit des Pumpvolumens mit der medizinischen Flüssigkeit wird die elastomere Membran auf die vorbeschriebene Weise ballonartig elastisch gedehnt und hierbei abschnittsweise von der dehnfesten Gitterstruktur zurückgehalten. Lediglich im Bereich der Gitteröffnungen bewirkt die elastische Dehnung der Membran ein Auswölben der Ausbuchtungsabschnitte unter Ausbildung jeweils einer der Ausbuchtungen. Die Gitteröffnungen der Gitterstruktur können regelmäßig oder unregelmäßig verteilt angeordnet sein. Die Gitteröffnungen können insbesondere rund, oval oder eckig gestaltet sein. Die Gitterstruktur kann als solche formstabil oder formnachgiebig und lediglich dehnfest gestaltet sein. Die Ausbuchtungsabschnitte können an der elastomeren Membran als solches vorgesehen und/oder erst durch ein Zusammenwirken mit der Gitterstruktur ausgebildet sein.

In weiterer Ausgestaltung der Erfindung sind die Gitteröffnungen unterschiedlich. Die elastische Dehnung der Membran bewirkt zusammen mit den unterschiedlich bemessenen Gitteröffnungen entsprechend unterschiedlich bemessene Ausbuchtungsabschnitte und letztlich Pumpvolumenabschnitte mit unterschiedlichem Volumeninhalt. Die Erfinder haben erkannt, dass eine solche Gestaltung im Hinblick auf die angestrebte möglichst konstante Förderrate der medizinischen Flüssigkeit weitere Vorteile bietet.

In weiterer Ausgestaltung der Erfindung ist die Gitterstruktur ein textiles Flächengebilde, wobei die Gitteröffnungen durch Maschen des textilen Flächengebildes gebildet sind. Das textile Flächengebilde ist vorzugsweise aus einem textilen Werkstoff gewirkt, gewebt, gestrickt oder geknüpft. Besonders vorteilhaft ist das textile Flächengebilde als Netz gestaltet. Das textile Flächengebilde ist unter Einwirkung der elastomeren Membran dehnfest. Durch die Gestaltung der Gitterstruktur als textiles Flächengebilde wird eine besonders kompakte Membrananordnung in einen Leerzustand der elastomeren Membran erreicht. Denn das textile Flächengebilde ist als solches formnachgiebig und kann zusammen mit der - nicht mit der medizinischen Flüssigkeit befüllten - elastomeren Membran zwecks Verpackung, Transport und/oder Lagerung kompakt zusammengelegt werden.

Die der Erfindung zugrunde liegende Aufgabe wird für die eingangs genannte medizinische Elastomerpumpe dadurch gelöst, dass eine elastomere Membran nach der vorstehenden Beschreibung und/oder eine Membrananordnung nach der vorstehenden Beschreibung vorgesehen ist.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine medizinische Elastomerpumpe nach dem Stand der Technik, die mit einer nach dem Stand der Technik gestalteten elastomeren Membran versehen und zur Förderung einer medizinischen Flüssigkeit im Rahmen einer Infusionstherapie vorgesehen ist,
- Fig. 2: eine Ausführungsform einer erfindungsgemäßen medizinischen Elastomerpumpe, die mit einer Ausführungsform einer erfindungsgemäßen elastomeren Membran versehen ist,
- Fig. 3: die elastomere Membran nach Fig. 2 in einer perspektivischen Detaildarstellung,
- Fig. 4: eine schematische Schnittdarstellung durch einen eben abgewickelten Membranabschnitt der elastomeren Membran nach Fig. 3,
- Fig. 5: eine Ausführungsform einer erfindungsgemäßen Membrananordnung mit einer elastomeren Membran und einer die elastomere Membran wenigstens abschnittsweise umhüllenden Gitterstruktur,
- Fig. 6: die Membrananordnung nach Fig. 5 in einer schematisch stark vereinfachten, abgeschnittenen und eben abgewickelten Querschnittsdarstellung, wobei die elastomere Membran einen Leerzustand einnimmt, und
- Fig. 7: die Membrananordnung nach Fig. 5 in einer der Fig. 6 entsprechenden Darstellungsweise, wobei die elastomere Membran einen wenigstens teilweise mit medizinischer Flüssigkeit befüllten Füllzustand einnimmt.

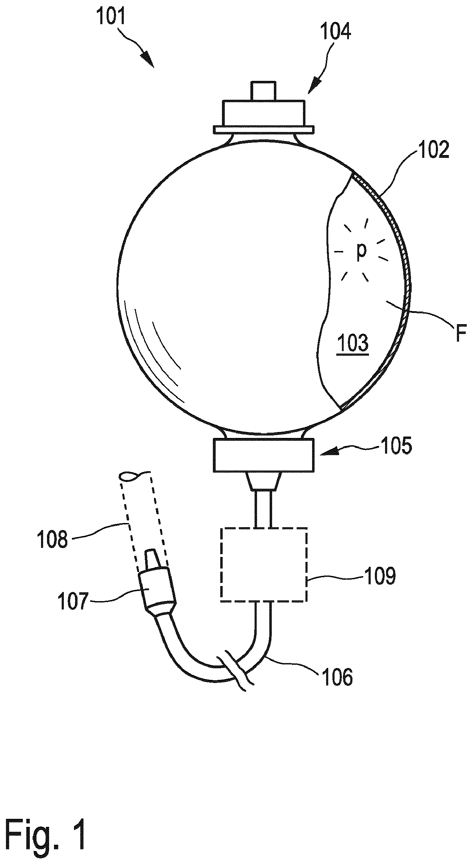

Gemäß Fig. 1 ist eine aus dem Stand der Technik bekannte medizinische Elastomerpumpe 101 zur Förderung einer medizinischen Flüssigkeit F im Rahmen einer ambulanten und/oder stationären Infusionstherapie vorgesehen. Die medizinische Elastomerpumpe 101 kann auch als elastomerische Infusionspumpe bezeichnet werden. Die medizinische Elastomerpumpe 101 weist eine elastomere Membran 102 auf, die ein Pumpvolumen 103 zur Aufnahme und Förderung der medizinischen Flüssigkeit F ausbildet. In der anhand Fig. 1 gezeigten Konfiguration ist das Pumpvolumen 103 in einem wenigstens teilweise mit der medizinischen Flüssigkeit F befüllten Füllzustand gezeigt. In diesem Füllzustand ist das Pumpvolumen 103 und damit auch die elastomere Membran 102 unter Einwirkung der medizinischen Flüssigkeit F ballonartig elastisch gedehnt. Die elastische Dehnung der Membran 102 bewirkt einen dehnungsabhängigen - und damit implizit füllstandsabhängigen - Förderdruck p auf das Pumpvolumen 103 und die darin aufgenommene medizinische Flüssigkeit F. Die aus dem Stand der Technik bekannte elastomere Membran 102 weist wenigstens im Füllzustand eine gleichmäßig rundlich ausgewölbte Gestalt auf, die vorliegend in etwa kugelförmig ausgebildet ist. Entsprechendes gilt für das von der elastomeren Membran 102 umgrenzte Pumpvolumen 103. Zum Befüllen des Pumpvolumens 103 mit der medizinischen Flüssigkeit F weist die medizinische Elastomerpumpe 101 einen wiederverschließbaren Einfüllstutzen 104 auf, der auf grundsätzlich bekannte Weise fluiddicht an die elastomere Membran 102 angeschlossen ist. Zur Ableitung der medizinischen Flüssigkeit F aus dem Pumpvolumen 103 weist die Elastomerpumpe 101 einen Auslassstutzen 105 auf, der auf grundsätzlich bekannte Weise fest und fluiddicht an die elastomere Membran 102 angeschlossen ist. In Bezug auf das Pumpvolumen 103 flussabwärts ist eine Schlauchleitung 106 einends fluidleitend mit dem Auslassstutzen 105 verbunden. Andernends ist die Schlauchleitung 106 auf grundsätzlich bekannte Weise mit einem Fluidkonnektor 107 versehen. Der Fluidkonnektor 107 ist in einem gebrauchsfertigen Zustand auf grundsätzlich bekannte Weise fluidleitend mit einem patientenseitigen Patientenzugang 108 verbunden, der anhand Fig. 1 lediglich strichliert angedeutet ist.

Aufgrund des dehnungs- damit füllzustandsabhängigen Förderdrucks p stellt sich naturgemäß eine nicht konstante Förderrate der medizinischen Flüssigkeit zwischen dem Pumpvolumen 103 und dem patientenseitigen Zugang 108 ein. Die Förderrate kann auch als Volumenstrom bezeichnet werden und ist aufgrund des dehnungsabhängigen Förderdrucks p letztlich über eine Förder- oder Verabreichungszeitdauer, während derer die medizinische Flüssigkeit F aus dem Pumpvolumen 103 gefördert wird, veränderlich. Bei der aus dem Stand der Technik bekannten Elastomerpumpe 101 ist ein Drosselelement 109 vorgesehen, das einer Verstetigung der Förderrate über der Förderdauer dienen soll. Das Drosselelement 109 ist anhand Fig. 1 lediglich schematisch angedeutet und auf grundsätzlich bekannte Weise der Schlauchleitung 106 zugeordnet. Das Drosselelement 109 kann beispielsweise in Form eines Druckminderers oder eines Flussratenbegrenzers gestaltet sein.

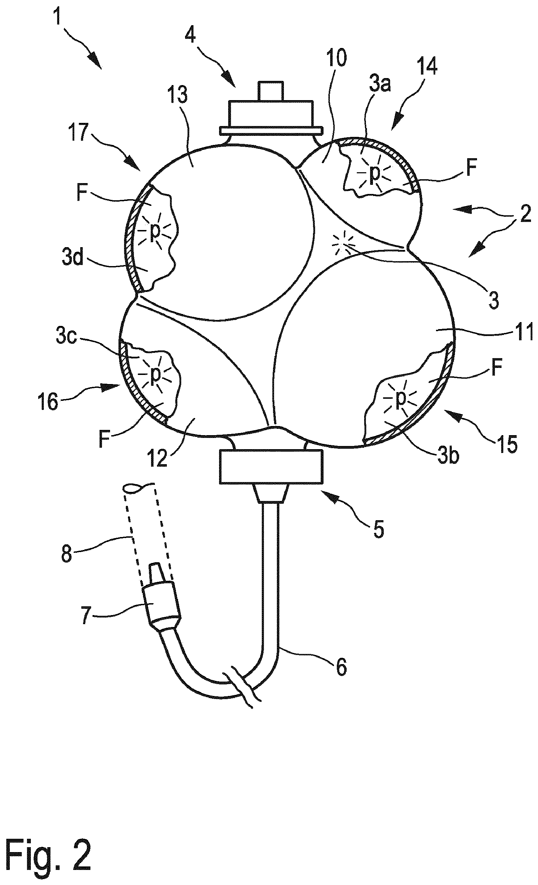

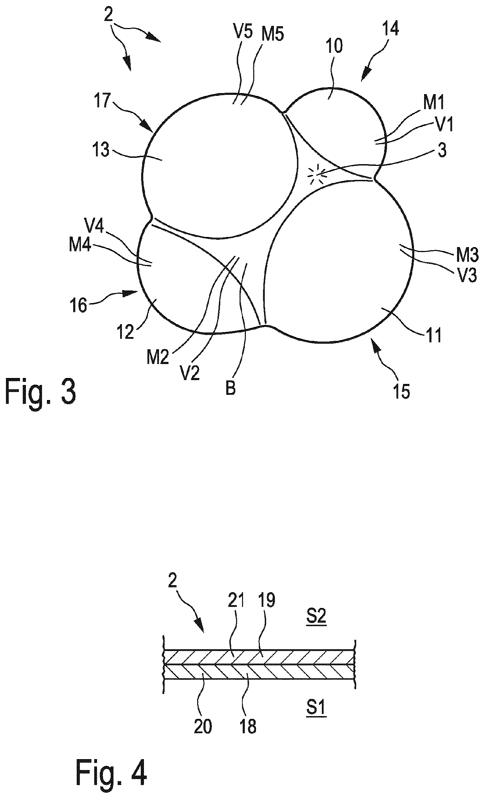

Gemäß Fig. 2 ist eine Ausführungsform einer erfindungsgemäßen medizinischen Elastomerpumpe 1 gezeigt, die in einer zu der aus dem Stand der Technik bekannten medizinischen Elastomerpumpe 101 nach Fig. 1 entsprechenden Weise zur Verabreichung einer medizinischen Flüssigkeit F vorgesehen ist. Dabei weist die medizinische Elastomerpumpe 1 im Unterschied zu der aus dem Stand der Technik bekannten medizinischen Elastomerpumpe 101 eine gemäß der Erfindung gestaltete elastomere Membran 2 auf. Die elastomere Membran 2 ist im Detail auch anhand Fig. 3 gezeigt.

Die elastomere Membran 2 weist mehrere Ausbuchtungsabschnitte 10, 11, 12, 13 auf. Anhand der Fig. 2 und 3 ist ein Füllzustand der elastomeren Membran 2 gezeigt, in welchem ein von der elastomeren Membran 2 ausgebildetes Pumpvolumen 3 zur Aufnahme und Förderung der medizinischen Flüssigkeit F wenigstens teilweise mit derselben befüllt ist. In diesem Füllzustand sind die Ausbuchtungsabschnitte 10, 11, 12, 13 der elastomeren Membran 2 unter Ausbildung jeweils einer Ausbuchtung 14, 15, 16, 17 rundlich ausgewölbt. Dabei bilden die Ausbuchtungen 14, 15, 16, 17 jeweils einen Pumpvolumenabschnitt 3a, 3b, 3c, 3d des Pumpvolumens 3 (Fig. 2).

Im Unterschied zu der aus dem Stand der Technik bekannten elastomeren Membran 102 nimmt die elastomere Membran 2 - wenigstens im Füllzustand - nicht etwa eine kugel-, ei- oder ballförmige Gestalt ein. Stattdessen ist die elastomere Membran 2 an mehreren Stellen unter Ausbildung der Ausbuchtungen14, 15, 16, 17 ausgerundet, ausgewölbt, geschwollen und/oder bauchig gestaltet. Die Ausbuchtungen 14, 15, 16, 17 sind gegenüber abseits der Ausbuchtungsabschnitte 10, 11, 12, 13 angeordneter Wandabschnitte der elastomeren Membran 2 in radialer Richtung nach außen ausgewölbt. Die Ausbuchtungen 14, 15, 16, 17 können jeweils insbesondere auch als Rundung, Wölbung, Schwellung, Bauch oder Buckel bezeichnet werden.

Bei der gezeigten Ausführungsform sind die Ausbuchtungen 14, 15, 16, 17 jeweils in Form einer Kugelkalotte gestaltet. Eine solche Gestaltgebung hat sich als vorteilhaft erwiesen. Bei einer nicht gezeigten Ausführungsform können die Ausbuchtungen abweichend von der hier gezeigten Kugelkalottenform gestaltet sein.

In einem zeichnerisch nicht näher gezeigten Leerzustand der elastomeren Membran 2, in welchem das Pumpvolumen 3 nicht mit der medizinischen Flüssigkeit F befüllt, sondern stattdessen leer ist, sind die Ausbuchtungen 14, 15, 16, 17 nicht oder in vergleichsweise geringerem Umfang ausgewölbt ausgebildet.

Vorliegend weist die elastomere Membran 2 insgesamt vier Ausbuchtungsabschnitte 10, 11, 12, 13 und dementsprechend im Füllzustand insgesamt vier Ausbuchtungen 14, 15, 16, 17 auf, was jedoch nicht zwingend ist. Bei einer nicht gezeigten Ausführungsform können weniger als vier Ausbuchtungsabschnitte und damit auch Ausbuchtungen vorhanden sein. Bei einer weiteren nicht gezeigten Ausführungsform können mehr als vier Ausbuchtungsabschnitte und damit auch Ausbuchtungen vorhanden sein.

Wie weiter anhand der Fig. 2 und 3 gezeigt ist, umfasst das von der elastomeren Membran 2 umgrenzte Pumpvolumen 3 die einzelnen Pumpvolumenabschnitte 3a, 3b, 3c, 3d. Der sich im Füllzustand einstellende Förderdruck p liegt innerhalb des gesamten Pumpvolumens 3 und damit auch in jedem einzelnen der Pumpvolumenabschnitte 3a, 3b, 3c, 3d an. Insoweit kann auch davon gesprochen werden, dass die Pumpvolumenabschnitte 3a, 3b, 3c, 3d - vereinfacht ausgedrückt - quasi zueinander parallel geschaltete Pumpvolumina oder auch Pumpen bilden.

Durch die erfindungsgemäße Gestaltung der elastomeren Membran 2 kann eine Verstetigung des Förderdrucks p über der elastischen Dehnung der elastomeren Membran 2 und somit letztlich eine möglichst konstante Förderrate der medizinischen Flüssigkeit F erreicht werden.

Der weitere Aufbau der medizinischen Elastomerpumpe 1 entspricht im Wesentlichen dem anhand Fig. 1 gezeigten und aus dem Stand der Technik bekannten Aufbau. Demgemäß weist die medizinische Elastomerpumpe 1 einen Einfüllstutzen 4 und einen Auslassstutzen 5 auf. Der Einlassstutzen 4 und der Auslassstutzen 5 sind auf grundsätzlich bekannte Weise fluiddicht an die elastomere Membran 2 angeschlossen. In der anhand Fig. 2 gezeigten Konfiguration ist auslassseitig eine Schlauchleitung 6 an den Auslassstutzen 5 angeschlossen, die an ihrem dem Auslassstutzen 5 abgewandten Stirnende mit einem Fluidkonnektor 7 versehen ist. Der Fluidkonnektor 7 ist zur fluidleitenden Verbindung mit einem schematisch angedeuteten patientenseitigen Patientenzugang 8 vorgesehen. Es versteht sich, dass auch bei der Elastomerpumpe 1 ein dem Drosselement 109 entsprechendes Drosselelement vorgesehen und der Schlauchleitung 6 zugeordnet sein kann.

Die elastomere Membran 2 weist - anders als Fig. 3 vermuten lässt - eine dem Einlassstutzen 4 zugeordnete Einlassöffnung und einem dem Auslassstutzen 5 zugeordnete Auslassöffnung auf, die jeweils auf grundsätzlich bekannte Weise in die elastomere Membran 2 eingebracht sein können und aus zeichnerischen Gründen in Fig. 3 nicht dargestellt sind.

Bei der gezeigten Ausführungsform weist die elastomere Membran 2 örtlich unterschiedliche elastische Dehnungseigenschaften auf. Dabei ist die elastomere Membran 2 im Bereich der Ausbuchtungsabschnitte 10, 11, 12, 13 vergleichsweise leichter elastisch dehnbar als in einem Bereich B abseits der Ausbuchtungsabschnitte 10, 11, 12, 13 (Fig. 3). Die örtlich unterschiedlichen Dehnungseigenschaften können durch örtlich unterschiedliche Werkstoffeigenschaften und/oder durch örtlich unterschiedliche Dimensionierungen der elastomeren Membran 2 bedingt sein. Dadurch, dass die elastomere Membran 2 im Bereich der Ausbuchtungsabschnitte 10, 11, 12, 13 vergleichsweise leichter elastisch dehnbar ist, wird ein zuverlässiges und funktionsgerechtes Auswölben unter Ausbildung der Ausbuchtungen 14, 15, 16, 17 gewährleistet.

Die örtlich unterschiedlichen elastischen Dehnungseigenschaften der elastomeren Membran 2 sind bei der gezeigten Ausführungsform durch unterschiedliche Membrandicken M1, M2 bedingt, wobei eine erste Membrandicke M1 im Bereich der Ausbuchtungsabschnitte 10, 11, 12, 13 und eine zweite Membrandicke M2 abseits der Ausbuchtungsabschnitte 10, 11, 12, 13 und somit insbesondere in dem Bereich B vorgesehen ist. Die erste Membrandicke M1 ist dabei vergleichsweise geringer als die zweite Membrandicke M2 bemessen. Dementsprechend ist die elastomere Membran 2 im Bereich der Ausbuchtungsabschnitte 10, 11, 12, 13 vergleichsweise leichter elastisch dehnbar.

Die erste Membrandicke M1 beträgt bei der gezeigten Ausführungsform 1,7 mm und die zweite Membrandicke M2 beträgt 1,9 mm. Grundsätzlich haben sich Membrandicken M1, M2 zwischen 0,5 mm und 4 mm und bevorzugt zwischen 1,5 mm und 2,5 mm als vorteilhaft erwiesen.

Die elastomere Membran 2 ist aus wenigstens einem elastomeren Werkstoff gefertigt. Hierfür kommen insbesondere Silikon in Form von Silikonkautschuk und/oder Silikonelastomer sowie Gummi infrage.

Weiter ist zur örtlich unterschiedlichen Ausgestaltung der Dehnungseigenschaften vorgesehen, dass die elastomere Membran 2, genauer: wenigstens ein elastomerer Werkstoff, aus welchem die elastomere Membran 2 gefertigt ist, örtlich unterschiedlich stark vernetzt ist. Dabei ist ein erster Vernetzungsgrad V1 im Bereich der Ausbuchtungsabschnitte 10, 11, 12, 13 und ein zweiter Vernetzungsgrad V2 abseits der Ausbuchtungsabschnitte 10, 11, 12, 13 und damit insbesondere auch in dem Bereich B vorgesehen. Der erste Vernetzungsgrad V1 ist vergleichsweise schwächer als der zweite Vernetzungsgrad V2. Hierdurch ist die elastomere Membran 2 im Bereich der Ausbuchtungsabschnitte 10, 11, 12, 13 vergleichsweise leichter elastisch dehnbar. Zur örtlich unterschiedlich starken Vernetzung kann ein entsprechendes Vulkanisationsverfahren angewendet werden. Solche Vulkanisationsverfahren sind als solche grundsätzlich bekannt. Vorliegend hat sich eine örtlich angepasste Vulkanisation mittels energiereicher Strahlung als besonders vorteilhaft erwiesen.

Bei der gezeigten Ausführungsform weist die elastomere Membran 2 nicht lediglich örtlich unterschiedliche elastische Dehnungseigenschaften im Hinblick auf die Ausbuchtungsabschnitte 10, 11, 12, 13 und den Bereich B abseits der Ausbuchtungsabschnitte 10, 11, 12, 13 auf (Fig. 3). Zusätzlich weisen auch die Ausbuchtungsabschnitte 10, 11, 12, 13 unterschiedliche elastische Dehnungseigenschaften auf. Hierdurch werden die Ausbuchtungsabschnitte 10, 11, 12, 13 unter Einwirkung des Förderdrucks p unterschiedlich ausgewölbt, so dass dementsprechend auch die Ausbuchtungen 14, 15, 16, 17 unterschiedlich sind. Die anhand der Fig. 2 und 3 ersichtliche unterschiedliche Ausbildung der Ausbuchtungen 14, 15, 16, 17 geht mit einem dementsprechend unterschiedlichen Volumeninhalt der Pumpvolumenabschnitte 3a, 3b, 3c, 3d einher. Vereinfacht ausgedrückt wird durch die insoweit unterschiedliche Gestaltung der Ausbuchtungsabschnitte 10, 11, 12, 13, der Ausbuchtungen 14, 15, 16, 17 und damit der Pumpvolumenabschnitte 3a, 3b, 3c, 3d eine Art Parallelschaltung unterschiedlicher Pumpen erreicht. Es hat sich gezeigt, dass dies im Hinblick auf die Verstetigung der Förderrate der medizinischen Flüssigkeit F weitere Vorteile bietet. Dennoch ist festzuhalten, dass die Ausbuchtungsabschnitte 10, 11, 12, 13 nicht zwingend unterschiedliche elastische Dehnungseigenschaften aufweise müssen. Dementsprechend sind die Ausbuchtungsabschnitte bei einer zeichnerisch nicht dargestellten Ausführungsform hinsichtlich ihrer elastischen Dehnungseigenschaften nicht unterschiedlich, sodass Pumpvolumenabschnitte gleichen Volumeninhalts erreicht werden.

Bei der gezeigten Ausführungsform werden die unterschiedlichen elastischen Dehnungseigenschaften der Ausbuchtungsabschnitte 10, 11, 12, 13 durch unterschiedliche Membrandicken M1, M3, M4, M5 erreicht. Der Ausbuchtungsabschnitt 10 weist die bereits vorgenannte erste Membrandicke M1 auf. Der Ausbuchtungsabschnitt 11 weist eine dritte Memrandicke M3 auf. Der Ausbuchtungsabschnitt 12 weist eine vierte Membrandicke M4 auf. Der Ausbuchtungsabschnitt 13 weist eine fünfte Membrandicke M5 auf. Die vorgenannten Membrandicken M1, M3, M4, M5 sind zueinander unterschiedlich bemessen und dementsprechend vergleichsweise dünner und/oder dicker.

Zur Ausbildung der unterschiedlichen elastischen Dehnungseigenschaften der Ausbuchtungsabschnitte 10, 11, 12, 13 ist vorliegend zusätlich eine unterschiedlich starke Vernetzung des elastomeren Werkstoffs der elastomeren Membran 2 im Bereich der Ausbuchtungsabschnitte 10, 11, 12, 13 vorgesehen. Dabei ist im Bereich des Ausbuchtungsabschnitts 10 der bereits vorgenannte erste Vernetzungsgrad V1 vorgesehen. Der Ausbuchtungsabschnitt 11 weist einen dritten Vernetzungsgrad V3 auf. Der Ausbuchtungsabschnitt 12 weist einen vierten Vernetzungsgrad V4 auf. Der Ausbuchtungsabschnitt 13 weist einen fünften Vernetzungsgrad V5 auf. Die vorgenannten Vernetzungsgrade V1, V3, V4, V5 sind zueinander vergleichsweise schwächer und/oder stärker.

Es versteht sich, dass die vorbeschriebenen Maßnahmen zum Erreichen der unterschiedlichen elastischen Dehnungseigenschaften der Ausbuchtungsabschnitte 10, 11, 12, 13 nicht zwingend in Kombination vorliegen müssen. Dementsprechend sind bei einer nicht gezeigten Ausführungsform hierzu lediglich unterschiedliche Membrandicken im Bereich der Ausbuchtungsabschnitte vorgesehen, wobei der elastomere Werkstoff im Bereich der Ausbuchtungsabschnitte ein und denselben Vernetzungsgrad aufweist. Bei einer weiteren nicht gezeigten Ausführungsform weisen die Ausbuchtungsabschnitte ein und dieselbe Membrandicke auf, wobei jedoch im Bereich der Ausbuchtungsabschnitte unterschiedliche Vernetzungsgrade des elastomeren Werkstoffs vorgesehen sind.

Die elastomere Membran 2 kann einschichtig oder mehrschichtig aufgebaut sein.

Bei der gezeigten Ausführungsform ist ein mehrschichtiger Membranaufbau mit einer ersten Membranschicht 18 und einer zweiten Membranschicht 19 vorgesehen (Fig. 4). Die erste Membranschicht 18 ist in radialer Richtung des Pumpvolumens 3 innenliegend und insoweit an einer Innenseite S1 der elastomeren Membran 2 angeordnet. Die zweite Membranschicht 19 ist in radialer Richtung außenliegend und somit an einer Außenseite S2 der elastomeren Membran 2 angeordnet. Die erste Membranschicht 10 ist vorliegend aus einem Silikonwerkstoff 20 gefertigt. Der Silikonwerkstoff 20 kann insbesondere ein Silikonkautschuk oder ein Silikonelastomer sein. Die erste Membranschicht 18 steht jedenfalls im Füllzustand in direktem Kontakt mit der medizinischen Flüssigkeit F. Dabei weist der Silikonwerkstoff 20 diesbezüglich vorteilhafte chemische Eigenschaften auf. Die zweite Membranschicht 19 ist bei der gezeigten Ausführungsform aus Gummi 21 gefertigt. Dabei dominiert die zweite Membranschicht 19 vorliegend die elastischen Dehnungseigenschaften der elastomeren Membran 2.

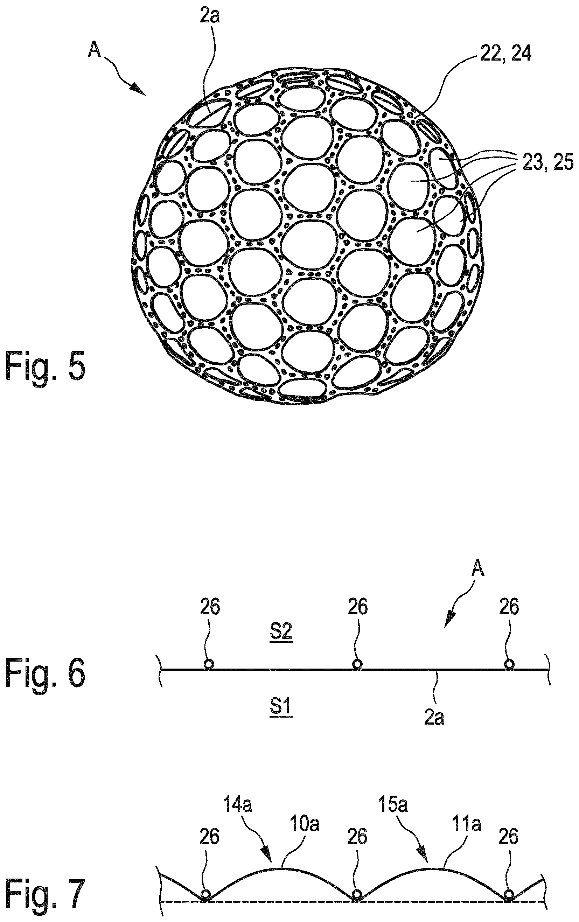

Gemäß Fig. 5 ist eine Membrananordnung A gezeigt, die eine elastomere Membran 2a und eine Gitterstruktur 22 aufweist. Die Membrananordnung A ist für eine medizinische Elastomerpumpe vorgesehen und kann beispielsweise anstelle der elastomeren Membran 2 bei der medizinischen Elastomerpumpe 1 nach Fig. 2 verwendet werden. Die elastomere Membran 2a bildet ein nicht näher bezeichnetes Pumpvolumen zur Aufnahme und Förderung der medizinischen Flüssigkeit F aus. Dabei ist die elastomere Membran 2a für sich alleine genommen wiederum ballonartig elastisch dehnbar. Allerdings ist die elastische Dehnbarkeit der elastomeren Membran 2a durch die Gitterstruktur 22 eingeschränkt. Die Gitterstruktur 22 ist dehnfest und umhüllt die elastomere Membran 2a wenigstens abschnittsweise. Dementsprechend ist die elastomere Membran 2a an ihrer Außenseite S2 in radialer Richtung an einer nicht näher bezeichneten Innenseite der Gitterstruktur 22 abgestützt. Die Gitterstruktur 22 weist mehrere Gitteröffnungen 23 auf. Wenigstens im Füllzustand der elastomeren Membran 2a ist diese im Bereich der Gitteröffnungen 23 unter Ausbildung jeweils einer Ausbuchtung 14a, 14b radial nach außen durch die Gitteröffnungen 23 hindurchgewölbt (Fig. 7). Die Gitteröffnungen 23 sind bei der gezeigten Ausführungsform etwa sechseckig gestaltet, was jedoch als rein exemplarisch zu verstehen ist.

Bei der anhand Fig. 5 gezeigten Ausführungsform sind die Gitteröffnungen 23 geringfügig unterschiedlich. Dies im Hinblick auf eine Formgebung und/oder einen Durchmesser der Gitteröffnungen 23. Bei einer nicht gezeigten Ausführungsform können diese Unterschiede stärker ausgeprägt sein. Bei einer weiteren nicht gezeigten Ausführungsform sind die Gitteröffnungen nicht unterschiedlich bemessen.

Die elastomere Membran 2a weist für sich alleine genommen keine spezifische Gestaltung zur Ausbildung der Ausbuchtungen 14a, 15a auf. Vielmehr werden entsprechende Ausbuchtungsabschnitte 10a, 11a gleichsam erst durch ein Zusammenwirken zwischen der elastomeren Membran 2a und der Gitterstruktur 22 ausgebildet.

Da die Gitteröffnungen 23 bei der gezeigten Ausführungsform unterschiedlich bemessen sind, werden die Ausbuchtungsabschnitte 10a, 11a dementsprechend unterschiedlich ausgebildet.

Anhand Fig. 5 ist die Membrananordnung A in einem Zustand gezeigt, in welchem die elastomere Membran 2a und damit deren Pumpvolumen nicht oder jedenfalls nicht nennenswert mit der medizinischen Flüssigkeit F befüllt sind. In einem solchen Leerzustand des Pumpvolumens liegt die Außenseite S2 der elastomeren Membran allenfalls lose an der Innenseite der Gitterstruktur 22 an oder ist gar von dieser beabstandet, was anhand Fig. 6 verdeutlicht ist. Erst in dem schematisch anhand Fig. 7 verdeutlichten Füllzustand sind die Ausbuchtungen 14a, 15a ausgebildet, wobei die entsprechenden Ausbuchtungsabschnitte 10a, 11a durch die Gitteröffnungen 23 hindurch rundlich ausgewölbt sind. Die Ausbuchtungen 14a, 15a sind in etwa kugelkalottenförmig ausgebildet. Dabei bildet jede der Ausbuchtungen 14a, 15a wiederum einen nicht näher bezeichneten Pumpvolumenabschnitt des Pumpvolumens aus. Durch die geringfügig unterschiedliche Gestaltung der Gitteröffnungen 23 sind, wie bereits erwähnt, die Ausbuchtungen 14a, 15a und der jeweils zugeordnete Pumpvolumenabschnitt - anders als dies Fig. 7 vermuten lässt - unterschiedlich. Auf eine entsprechende zeichnerische Verdeutlichung wird der Einfachheit halber verzichtet. Infolge der unterschiedlichen Gitteröffnungen 23 kann beispielsweise die Ausbuchtung 14a stärker ausgeewölbt sein als die Ausbuchtung 15a oder umgekehrt. Entsprechendes gilt für den jeweils zugeodneten Pumpvolumenabschnitt. Die Gitterstruktur 22 ist dehnfest und verbleibt somit auch bei einem Befüllen der elastomeren Membran 2a mit der medizinischen Flüssigkeit F in der anhand Fig. 5 ersichtlichen Konfiguration. Insoweit wird die Gitterstruktur 22 nicht etwa ballonartig elastisch gedehnt.

Die Gitterstruktur 22 ist bei der gezeigten Ausführungsform als textiles Flächengebilde 24 gestaltet. Das textile Flächengebilde 24 ist in Form eines grobmaschigen Netzes gefertigt, wobei die Gitteröffnungen 23 durch Maschen 25 des textilen Flächengebildes 24 gebildet sind. Das textile Flächengebilde 24 kann insbesondere gewirkt, gestrickt, gewebt oder geknüpft sein und weist insoweit miteinander wirkverbundene Textilstränge 26 auf, welche die Maschen 25 bilden oder - mit anderen Worten ausgedrückt - die Gitteröffnungen 23 umgrenzen (Fig. 6, 7).

Bei einer nicht gezeigten Ausführungsform ist die Gitterstruktur aus einem formstabilen Werkstoff gefertigt. Insbesondere kann die Gitterstruktur aus Metall oder Kunststoff gefertigt sein.

## Patentansprüche

1. Elastomere Membran (2) für eine medizinische Elastomerpumpe (1) zur Förderung einer medizinischen Flüssigkeit (F), wobei die elastomere Membran (2) ein Pumpvolumen (3) zur Aufnahme und Förderung der medizinischen Flüssigkeit (F) ausbildet und in einem wenigstens teilweise mit der medizinischen Flüssigkeit (F) befüllten Füllzustand des Pumpvolumens (3) elastisch gedehnt ist, wodurch die elastisch gedehnte elastomere Membran (2) einen Förderdruck (p) auf das Pumpvolumen (3) zur Förderung der medizinischen Flüssigkeit (F) bewirkt, **dadurch gekennzeichnet, dass** die elastomere Membran (2) mehrere Ausbuchtungsabschnitte (10, 11, 12, 13) aufweist, die wenigstens im Füllzustand unter Ausbildung jeweils einer Ausbuchtung (14, 15, 16, 17) ausgewölbt sind, wobei die Ausbuchtungen (14, 15, 16, 17) jeweils einen Pumpvolumenabschnitt (3a, 3b, 3c, 3d) des Pumpvolumens (3) bilden.

2. Elastomere Membran (2) nach Anspruch 1, **gekennzeichnet durch** örtlich unterschiedliche elastische Dehnungseigenschaften, wobei die elastomere Membran (2) im Bereich der Ausbuchtungsabschnitte (10, 11, 12, 13) vergleichsweise leichter elastisch dehnbar ist als abseits der Ausbuchtungsabschnitte (10, 11, 12, 13).

3. Elastomere Membran (2) nach Anspruch 1 oder 2, **dadurch gekennzeichnet, dass** die Ausbuchtungsabschnitte (10, 11, 12, 13) unterschiedliche elastische Dehnungseigenschaften aufweisen, wodurch die Ausbuchtungen (14, 15, 16, 17) unter Einwirkung des Förderdrucks (p) unterschiedlich ausgebildet sind.

4. Elastomere Membran (2) nach einem der vorhergehenden Ansprüche, **gekennzeichnet durch** örtlich unterschiedliche Membrandicken (M1, M2, M3, M4, M5), wobei eine erste Membrandicke (M1) im Bereich der Ausbuchtungsabschnitte (10, 11, 12, 13) vergleichsweise geringer ist als eine zweite Membrandicke (M2) abseits der Ausbuchtungsabschnitte (10, 11, 12, 13).

5. Elastomere Membran (2) nach Anspruch 4, **dadurch gekennzeichnet, dass** die Ausbuchtungsabschnitte (10, 11, 12, 13) unterschiedliche Membrandicken (M1, M3, M4, M5) aufweisen.

6. Elastomere Membran (2) nach einem der vorhergehenden Ansprüche, **gekennzeichnet durch** eine Fertigung aus wenigstens einem elastomeren Werkstoff (20, 21), der örtlich unterschiedlich stark vernetzt ist, wobei ein erster Vernetzungsgrad (V1) im Bereich der Ausbuchtungsabschnitte (10, 11, 12, 13) vergleichsweise schwächer ist als ein zweiter Vernetzungsgrad (V2) abseits der Ausbuchtungsabschnitte (10, 11, 12, 13).

7. Elastomere Membran (2) nach Anspruch 6, **dadurch gekennzeichnet, dass** im Bereich der Ausbuchtungsabschnitte (10, 11, 12, 13) unterschiedliche Vernetzungsgrade (V1, V3, V4, V5) vorgesehen sind.

8. Elastomere Membran (2) nach einem der vorhergehenden Ansprüche, **dadurch gekennzeichnet, dass** zwischen 2 und 100, bevorzugt zwischen 7 und 40, besonders bevorzugt zwischen 15 und 25, Ausbuchtungsabschnitte (10, 11, 12, 13) vorgesehen sind.

9. Elastomere Membran (2) nach einem der vorhergehenden Ansprüche, **dadurch gekennzeichnet, dass** eine Membrandicke und/oder örtlich unterschiedliche Membrandicken (M1, M2, M3, M4, M5) zwischen 0,5 mm und 4 mm, bevorzugt zwischen 1,5 mm und 2,5 mm, besonders bevorzugt zwischen 1,7 mm und 1,9 mm, vorgesehen sind.

10. Elastomere Membran (2) nach einem der vorhergehenden Ansprüche, **gekennzeichnet durch** einen wenigstens einschichtigen Membranaufbau, der wenigstens eine aus Silikon (20) gefertigte erste Membranschicht (18) aufweist.

11. Elastomere Membran (2) nach Anspruch 10, **gekennzeichnet durch** einen mehrschichtigen Membranaufbau, der wenigstens eine aus Gummi (21) gefertigte zweite Membranschicht (19) aufweist.

12. Membrananordnung (A) für eine medizinische Elastomerpumpe (1) zur Förderung einer medizinischen Flüssigkeit (F), mit einer elastomeren Membran (2, 2a) nach einem der vorhergehenden Ansprüche oder nach dem Oberbegriff von Anspruch 1 und mit einer die elastomere Membran (2, 2a) wenigstens abschnittsweise umhüllenden dehnfesten Gitterstruktur (22), an deren radial innenliegender Innenseite die elastomere Membran (2, 2a) wenigstens im Füllzustand radial nach außen abgestützt ist, wobei die Gitterstruktur (22) mehrere Gitteröffnungen (23) aufweist, durch welche die elastomere Membran (2, 2a) wenigstens im Füllzustand unter Ausbildung jeweils einer Ausbuchtung (14a, 15a) radial ausgewölbt ist.

13. Membrananordnung (A) nach Anspruch 12, **dadurch gekennzeichnet, dass** die Gitteröffnungen (23) unterschiedlich sind.

14. Membrananordnung (A) nach Anspruch 12 oder 13, **dadurch gekennzeichnet, dass** die Gitterstruktur (22) ein textiles Flächengebilde (24) ist, wobei die Gitteröffnungen (23) durch Maschen (25) des textilen Flächengebildes (24) gebildet sind.

15. Medizinische Elastomerpumpe (1) zur Förderung einer medizinischen Flüssigkeit (F) mit einer elastomeren Membran (2) nach einem der Ansprüche 1 bis 11 und/oder einer Membrananordnung (A) nach einem der Ansprüche 12 bis 14.

## Claims

1. An elastomeric membrane (2) for a medical elastomer pump (1) for conveying a medical fluid (F), wherein the elastomeric membrane (2) forms a pump volume (3) for receiving and conveying the medical fluid (F) and is elastically stretched in a filled state of the pump volume (3) at least partially filled with the medical fluid (F), as a result of which the elastically stretched elastomeric membrane (2) exerts a conveying pressure (p) on the pump volume (3) so as to convey the medical fluid (F), **characterized in that** the elastomeric membrane (2) has a plurality of bulge portions (10, 11, 12, 13) that, at least in the filled state, curve outward so as to each form a bulge (14, 15, 16, 17), wherein the bulges (14, 15, 16, 17) each form a pump volume portion (3a, 3b, 3c, 3d) of the pump volume (3).

2. The elastomeric membrane (2) as claimed in claim 1, **characterized by** locally different elastic stretch properties, wherein the elastomeric membrane (2) is elastically stretchable comparatively more easily in the region of the bulge portions (10, 11, 12, 13) than away from the bulge portions (10, 11, 12, 13).

3. The elastomeric membrane (2) as claimed in claim 1 or 2, **characterized in that** the bulge portions (10, 11, 12, 13) have different elastic stretch properties, as a result of which the bulges (14, 15, 16, 17) are formed differently under the effect of the conveying pressure (p).

4. The elastomeric membrane (2) as claimed in any of the preceding claims, **characterized by** locally different membrane thicknesses (M1, M2, M3, M4, M5), wherein a first membrane thickness (M1) in the region of the bulge portions (10, 11, 12, 13) is comparatively smaller than a second membrane thickness (M2) away from the bulge portions (10, 11, 12, 13).

5. The elastomeric membrane (2) as claimed in claim 4, **characterized in that** the bulge portions (10, 11, 12, 13) have different membrane thicknesses (M1, M3, M4, M5).

6. The elastomeric membrane (2) as claimed in any of the preceding claims, **characterized by** manufacture from at least one elastomeric material (20, 21) that is crosslinked to locally differing degrees, wherein a first degree of crosslinking (V1) in the region of the bulge portions (10, 11, 12, 13) is comparatively weaker than a second degree of crosslinking (V2) away from the bulge portions (10, 11, 12, 13).

7. The elastomeric membrane (2) as claimed in claim 6, **characterized in that** different degrees of crosslinking (V1, V3, V4, V5) are provided in the region of the bulge portions (10, 11, 12, 13).

8. The elastomeric membrane (2) as claimed in any of the preceding claims, **characterized in that** between 2 and 100, preferably between 7 and 40, particularly preferably between 15 and 25, bulge portions (10, 11, 12, 13) are provided.

9. The elastomeric membrane (2) as claimed in any of the preceding claims, **characterized in that** a membrane thickness and/or locally different membrane thicknesses (M1, M2, M3, M4, M5) between 0.5 mm and 4 mm, preferably between 1.5 mm and 2.5 mm, particularly preferably between 1.7 mm and 1.9 mm, is/are provided.

10. The elastomeric membrane (2) as claimed in any of the preceding claims, **characterized by** an at least single-layer membrane construction that has at least one first membrane layer (18) manufactured from silicone (20).

11. The elastomeric membrane (2) as claimed in claim 10, **characterized by** a multilayer membrane construction that has at least one second membrane layer (19) manufactured from rubber (21).

12. A membrane arrangement (A) for a medical elastomer pump (1) for conveying a medical fluid (F), having an elastomeric membrane (2, 2a) as claimed in any of the preceding claims or according to the preamble of claim 1 and having a stretch-resistant lattice structure (22) that envelops the elastomeric membrane (2, 2a) at least in certain portions, the elastomeric membrane (2, 2a) being radially outwardly supported on the radially inner interior side of said lattice structure at least in the filled state, wherein the lattice structure (22) has a plurality of lattice openings (23), through which the elastomeric membrane (2, 2a), at least in the filled state, radially curves outward so as to form in each case a bulge (14a, 15a).

13. The membrane arrangement (A) as claimed in claim 12, **characterized in that** the lattice openings (23) are different.

14. The membrane arrangement (A) as claimed in claim 12 or 13, **characterized in that** the lattice structure (22) is a textile fabric (24), wherein the lattice openings (23) are formed by meshes (25) of the textile fabric (24).

15. A medical elastomer pump (1) for conveying a medical fluid (F) having an elastomeric membrane (2) as claimed in any of claims 1 to 11 and/or a membrane arrangement (A) as claimed in any of claims 12 to 14.

## Revendications

1. Membrane élastomère (2) pour une pompe élastomère médicale (1) pour transporter un liquide médical (F), la membrane élastomère (2) réalisant un volume de pompage (3) pour recevoir et transporter le liquide médical (F) et étant étirée élastiquement dans un état de remplissage du volume de pompage (3) au moins partiellement rempli avec le liquide médical (F), moyennant quoi la membrane élastomère étirée élastiquement (2) exerce une pression de transport (p) sur le volume de pompage (3) pour transporter le liquide médical (F), **caractérisée en ce que** la membrane élastomère (2) présente plusieurs sections de renflement (10, 11, 12, 13) qui, au moins à l'état de remplissage, sont bombées en réalisant chacune un renflement (14, 15, 16, 17), les renflements (14, 15, 16, 17) formant chacun une section de volume de pompage (3a, 3b, 3c, 3d) du volume de pompage (3).

2. Membrane élastomère (2) selon la revendication 1, **caractérisée par** des propriétés d'extension élastique localement différentes, la membrane élastomère (2) étant comparativement plus facilement extensible élastiquement dans la zone des sections de renflement (10, 11, 12, 13) qu'à l'écart des sections de renflement (10, 11, 12, 13).

3. Membrane élastomère (2) selon la revendication 1 ou 2, **caractérisée en ce que** les sections de renflement (10, 11, 12, 13) présentent des propriétés d'extension élastique différentes, moyennant quoi les renflements (14, 15, 16, 17) sont réalisés différemment sous l'effet de la pression de transport (p).

4. Membrane élastomère (2) selon l'une quelconque des revendications précédentes, **caractérisée par** des épaisseurs de membrane (M1, M2, M3, M4, M5) localement différentes, une première épaisseur de membrane (M1) dans la zone des sections de renflement (10, 11, 12, 13) étant comparativement plus faible qu'une deuxième épaisseur de membrane (M2) à l'écart des sections de renflement (10, 11, 12, 13).

5. Membrane élastomère (2) selon la revendication 4, **caractérisée en ce que** les sections de renflement (10, 11, 12, 13) présentent des épaisseurs de membrane différentes (M1, M3, M4, M5).

6. Membrane élastomère (2) selon l'une quelconque des revendications précédentes, **caractérisée par** une fabrication à partir d'au moins un matériau élastomère (20, 21) réticulé localement à des degrés différents, un premier degré de réticulation (V1) dans la zone des sections de renflement (10, 11, 12, 13) étant comparativement plus faible qu'un deuxième degré de réticulation (V2) à l'écart des sections de renflement (10, 11, 12, 13).

7. Membrane élastomère (2) selon la revendication 6, **caractérisée en ce que** différents degrés de réticulation (V1, V3, V4, V5) sont prévus dans la zone des sections de renflement (10, 11, 12, 13).

8. Membrane élastomère (2) selon l'une quelconque des revendications précédentes, **caractérisée en ce qu'**entre 2 et 100, de préférence entre 7 et 40, de manière particulièrement préférée entre 15 et 25 sections de renflement (10, 11, 12, 13) sont prévues.

9. Membrane élastomère (2) selon l'une quelconque des revendications précédentes, **caractérisée en ce qu'**une épaisseur de membrane et/ou des épaisseurs de membrane localement différentes (M1, M2, M3, M4, M5) comprises entre 0,5 mm et 4 mm, de préférence entre 1,5 mm et 2,5 mm, de manière particulièrement préférée entre 1,7 mm et 1,9 mm, sont prévues.

10. Membrane élastomère (2) selon l'une quelconque des revendications précédentes, **caractérisée par** une structure de membrane au moins monocouche, présentant au moins une première couche de membrane (18) fabriquée en silicone (20).

11. Membrane élastomère (2) selon la revendication 10, **caractérisée par** une structure de membrane multicouche présentant au moins une deuxième couche de membrane (19) fabriquée en caoutchouc (21).

12. Agencement de membrane (A) pour une pompe élastomère médicale (1) pour le transport d'un liquide médical (F), avec une membrane élastomère (2, 2a) selon l'une quelconque des revendications précédentes ou selon le terme générique de la revendication 1 et avec une structure de grille (22) résistante à l'extension enveloppant au moins par sections la membrane élastomère (2, 2a), sur le côté intérieur radialement intérieur de laquelle la membrane élastomère (2, 2a) s'appuie radialement vers l'extérieur au moins à l'état de remplissage, la structure de grille (22) présentant plusieurs ouvertures de grille (23) à travers lesquelles la membrane élastomère (2, 2a) est bombée radialement au moins à l'état de remplissage en réalisant respectivement un renflement (14a, 15a).

13. Agencement de membrane (A) selon la revendication 12, **caractérisé en ce que** les ouvertures de grille (23) sont différentes.

14. Agencement de membrane (A) selon la revendication 12 ou 13, **caractérisé en ce que** la structure de grille (22) est une structure textile plane (24), les ouvertures de grille (23) étant formées par des mailles (25) de la structure textile plane (24).

15. Pompe élastomère médicale (1) pour le transport d'un fluide médical (F) avec une membrane élastomère (2) selon l'une quelconque des revendications 1 à 11 et/ou un agencement de membrane (A) selon l'une quelconque des revendications 12 à 14.
